# EUROPEAN PATENT APPLICATION

(11) **EP 0 608 779 A1**
(43) Date of publication of application: **03.08.1994**
(21) Application number: 94100864.1
(22) Date of filing: 21.01.1994
(51) Int. Cl.: B01J 19/00, C07K 1/04

(54) **Apparatus for peptide synthesis**

(30) Priority: 23.01.1993 JP 27641/93
(71) Applicant: SHIMADZU CORPORATION, Nakagyo-ku Kyoto-shi Kyoto 604 (JP)
(72) Inventor: Nokihara, Kiyoshi, c/o SHIMADZU CORPORATION, Kyoto-shi, Kyoto 604 (JP)
(74) Representative: WILHELMS, KILIAN & PARTNER Patentanwälte

(57) **Abstract**

An apparatus for peptide synthesis of the present invention has a means of repeating a series of treatments consisting of washing, coupling, washing and deprotecting in a stream of an inert gas; a needle for injection /aspiration of reagents; and a microtiter plate with wells of which inner wall has a linker or a microtiter plate having a membrane filter, which has a linker, placed in its wells. The peptide synthesizer allows easy washing of the solid support with only a small amount of solvent. It also makes it possible to get the synthesized product as a peptide (or DNA) solution directly from the reaction vessel by sequentially performing cleavage in the reaction vessel (in situ cleavage) after the completion of peptide chain-assembly.

## Description

### BACKGROUND OF THE INVENTION

### Field of the Invention

The present invention relates to an apparatus for peptide synthesis which uses a microtiter plate having wells with a derivatized inner wall or a microtiter plate having a derivatized membrane filter placed in its wells.

### Discussion of the Related Art

Generally, synthesis of DNA, RNA or peptides (hereinafter simply referred to as peptide synthesis) has been achieved by repeating a series of treatments of washing, coupling, washing and deprotection on a solid support of glass beads or resin powder in a reaction vessel. When a conventional method for peptide synthesis is used, resin washing is key to prevent contamination. However, complete washing of a resin support in the reaction vessel with a solvent such as methylene chloride, DMF(dimethylformamide) and acetonitrile consumes a very large amount of solvent and much time. Also, when the peptide is cleaved from the peptidyl resin, a separate procedure to cleave the resin-peptide bond is required. These two requirements are obstacles to a rapid and efficient peptide synthesis, and there has been a strong need for improvement.

### SUMMARY OF THE INVENTION

The object of the present invention is to provide an apparatus for peptide synthesis, which allows easy washing of a solid support with a small amount of washing solvent; and cleavage of peptide-resin bond in the reaction vessel (in situ cleavage) as an integrated treatment following the completion of peptide elongation, which makes it possible to obtain the desired peptide (or DNA) dissolved in a solvent directly from the reaction vessel.

The inventor of the present invention has made an extensive study to achieve the above object, and has eventually found it very useful to use a microtiter plate having wells with a derivatized inner wall or a microtiter plate having a derivatized membrane filter placed in its wells. After further developmental work, the inventor has completed the present invention.

In summary, the present invention relates to an apparatus for peptide synthesis having a means of repeating a series of treatments consisting of washing, coupling, washing and deprotecting in a stream of an inert gas; a needle for injection /aspiration of reagents; and a microtiter plate with wells of which inner wall has a linker (hereinafter simply referred to as "derivatized inner wall") or a microtiter plate having a membrane filter, which has a linker (hereinafter simply referred to as "derivatized membrane filter"), placed in its wells.

Specifically, the term "derivatized" used in the present specification means "to be chemically treated to have a linker."

The peptide synthesizer of the present invention allows easy washing of the solid support with only a small amount of solvent. It also makes it possible to get the synthesized product as a peptide (or DNA) solution directly from the reaction vessel by sequentially performing cleavage in the reaction vessel (in situ cleavage) after the completion of peptide chain-assembly.

Also, when a linker used is such that it is hardly cleaved under mild conditions using reagents such as neat TFA(trifluoroacetic acid), the microtiter plate can be used as a substrate for a reaction based on molecular recognition such as an antigen for antigen-antibody reactions in immunoassay (ELISA), etc., while the peptide thus synthesized is immobilized on the derivatized inner wall of the microtiter plate or the derivatized membrane filter in the microtiter plate.

Moreover, the peptide synthesizer of the present invention can be produced at low cost and allows rapid, highly efficient, parallel or simultaneous synthesis of a plurality of peptides at low running cost.

### BRIEF DESCRIPTION OF THE DRAWINGS

The present invention will become more fully understood from the detailed description given by way of illustration only, and thus, are not limitative of the present invention and wherein:
Figure 1 is a schematic view showing the relationship of the reagent bottles, needle and microtiter plate in an apparatus for peptide synthesis of the present invention; and
Figure 2 is a perspective view showing a part of the microtiter plate used in an apparatus for peptide synthesis of the present invention, with a well having a derivatized membrane filter and a needle.

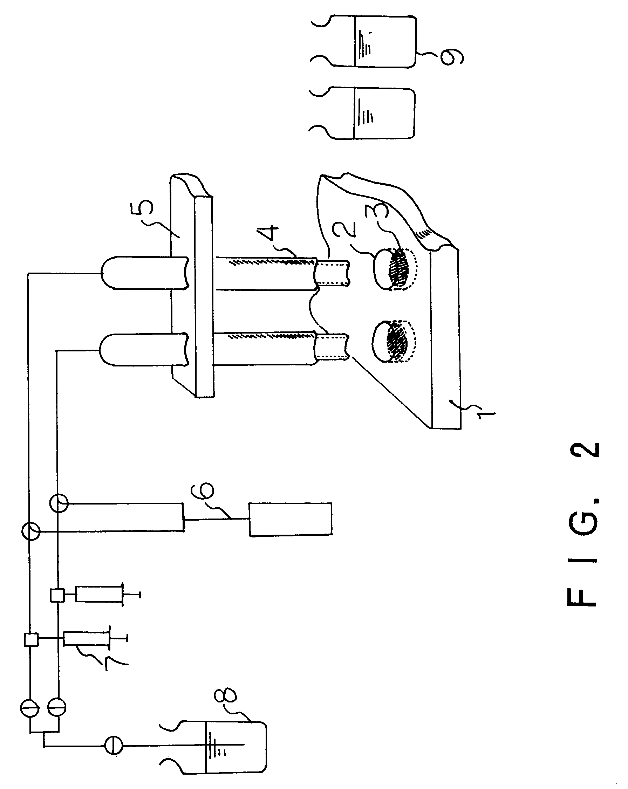

The reference numerals in Figure 2 denote the following elements:
Element 1 is a microtiter plate; element 2 a well of a microtiter plate; element 3 a membrane filter; element 4 a needle for injection/aspiration of reagent; element 5 an arm; element 6 a gas supply line; element 7 an injection /aspiration line; element 8 a bottle for washing solvent; and element 9 a reagent bottle.

### DETAILED DESCRIPTION OF THE INVENTION

In the present invention, the term "cleavage" is defined as the operation for liberation of the desired peptide from the solid support generated in a solid-phase peptide synthesizer. The linkers suitable for cleavage are exemplified by acid labile linkers/handles such as hydroxyphenoxyacetic acid, 4-hydroxymethylbenzoic acid, 4-(4-hydroxymethyl-3-methoxyphenoxy)-butyric acid, 4-hydroxymethylphenoxyacetic acid, 3-(4-hydroxymethylphenoxy) propionic acid, 2,4-dimethoxy-4'-hydroxy-benzophenone and p-[(R,S)-α-[1-(9H-fluoren-9-yl)-methoxyformamido]-2,4-dimethoxybenzyl]-phenoxyacetic acid. As described above, in the present invention, to chemically treat the solid support to have such a linker is simply referred to as "to derivatize."

In the present invention, a microtiter plate having a well with derivatized inner wall can be prepared by chemically treating the inner wall of each well which is made of a plastic material such as polypropylene, polyethylene and polystyrene, or by coating the inner wall of each well with an easy-to-derivatize substance such as polyhydroxysuccine imide. Specifically, the inner wall of each well is derivatized by introducing a linker to the functional group on the wall such as a hydroxyl group when the inner wall of the well is made of cellulose, paper or cotton, or an aminoethyl group when the inner wall of the well is made of a polymer. Then, the first protected amino acid is bound to the linker. All these procedures are well known methods which are widely used in the field of peptide synthesis. When the inner wall of the well is made of a polymer, a spacer which does not interfere with peptide chain elongation can be introduced between the inner wall and the linker by conventional methods. Such spacer is exemplified by polyoxyethylene(POE).

A derivatized membrane filter of polypropylene, polyvinylidene difluoride, cellulose, glass or another material can be prepared by conventional chemical treatments, and pieces, which fit to the microtiter plate wells in size, are cut out from the derivatized membrane. In this case, the piece is preferably cut out to have a diameter slightly larger (larger by about 0.5 mm) than that of the well to get a tight fit in the well. Each piece is put into each well to prepare a microtiter plate having a derivatized membrane filter placed in its wells. It is preferred to tailor a special cutter for this purpose, by which pieces of the derivatized membrane filter, which fit to the wells in size and number, can be cut out all at once and sequentially be placed down into the wells.

The peptide synthesizer of the present invention is hereinafter described in detail.

The peptide synthesizer of the present invention is for a step-wise reaction, and used for the preparation of a peptide library or a DNA library (primer). It can synthesize a plurality of peptides concurrently because it uses a microtiter plate having multiple wells. Also, the apparatus of peptide synthesis of the present invention has a means for repeating a series of treatments of washing, coupling, washing and deprotection in an inert gas stream and a needle which injects or aspirates a reagent for its delivery or discard. Here, such repeating for the synthesis of DNA is preferably carried out under closed conditions.

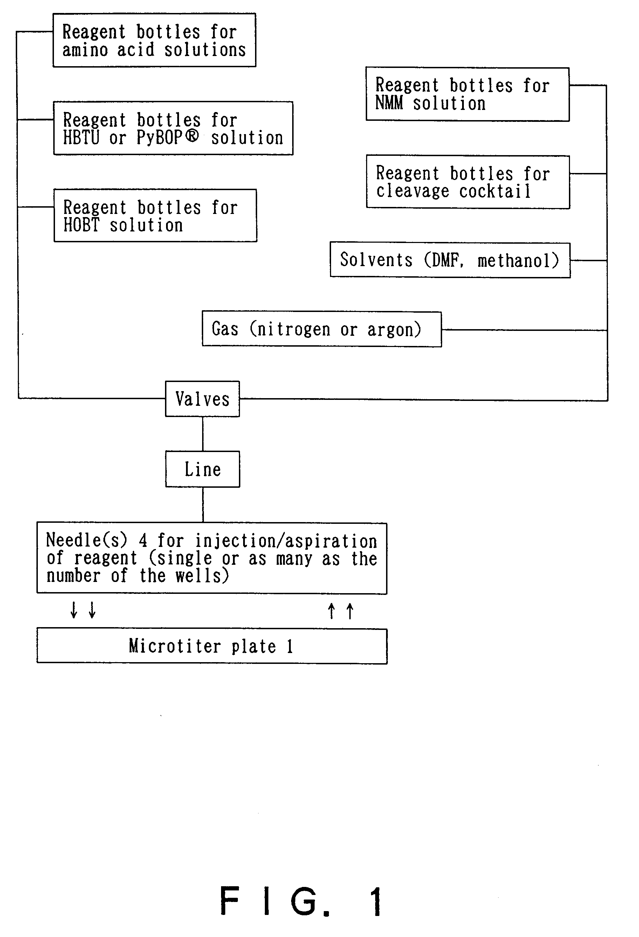

Referring to Figures 1 and 2, the peptide synthesizer of the present invention and its structural details, particularly relationship between needle behavior and microtiter plate, are described below.

Figure 1 is a schematic diagram showing the relationships among various reagent bottles, needles and a microtiter plate in a peptide synthesizer of the present invention. Figure 2 is a perspective view showing the needle and microtiter plate portions in a peptide synthesizer of the present invention.

A microtiter plate 1, having a membrane filter 3 placed in each well 2 thereof, is installed in the peptide synthesizer. A needle 4 for injection/aspiration of reagent (hereinafter simply referred to as needle) behaves over the well 2 in the same manner as, for example, Pipetting Robot manufactured by Techan Company. The number of needles is determined according to necessity. The needle 4 is held in the peptide synthesizer by an arm 5 which is arranged to allow the needle to move horizontally and vertically.

The needle 4 is connected to an injection /aspiration line 7 for delivering a reaction reagent such as amino acids, solvents and cleavage cocktail to well 2 or discarding a reaction mixture from well 2. Also, a gas supply line 6 for supplying an inert gas to the wells, where a series of treatments of washing, coupling, washing and deprotection is repeated in an inert gas stream, connects the needle portion to a gas supply portion.

Peptide synthesis by the peptide synthesizer of the present invention is achieved using a microtiter plate having wells with a derivatized inner wall or a microtiter plate having a derivatized membrane filter placed in its wells. Peptide synthesis proceeds on the derivatized inner wall or the derivatized membrane filter, while the needle 4 as shown in Figures 1 and 2 repeats injection and aspiration of a reaction solution to deliver the solution to the well 2 and discard it from the well 2 after completion of each reaction. During the synthesis, a series of treatments of washing, coupling, washing and deprotection is repeated as necessary, while an inert gas is supplied to the reaction vessel. A synthesized peptide, which is bound to the inner wall of the well or the membrane filter placed in the well of the microtiter plate, can be treated in each well by an ordinary method for cleavage (in situ cleavage) to yield a solution containing the desired peptide, from which the desired peptide can be purified by conventional methods such as HPLC.

Also, when phenylacetamidomethyl-derivatized polymer is used as a linker, since this linker is hardly cleaved under mild conditions using reagents such as neat TFA, the microtiter plate can be used as a substrate for a reaction based on molecular recognition such as an antigen for antigen-antibody reaction in immunoassay(ELISA), while the peptide thus synthesized is immobilized on the derivatized inner wall of the microtiter plate or the derivatized membrane filter in the microtiter plate. The embodiment using such kind of linkers is also within the scope of the present invention.

### EXAMPLE

The present invention is hereinafter described in more detail by means of the following working examples, but the present invention is not limited by them.

### Example 1: Preparation of a microtiter plate having wells with a derivatized inner wall

A 96-well microtiter plate is prepared. As methods for titer plate processing with polymer, the method using a polystyrene-polyethylene graft polymer is available (e.g., R.H. Berg et al., Peptides, 1988, E. Bayer and G. Jung eds., Walter de Gruyter & Co. Berlin-New York 1989, pp. 196-198; R.H. Berg et al., J. Am. Chem. Soc. 111, 8024-8029, 1989). In this Example 1, a polymer of polypropylene is prepared and formed into a titer plate. Functional groups are then introduced to the inner wall of the titer plate so that the amino functional group of the aminoethyl group is exposed, by the method described in the above-mentioned publications. Hydroxyphenoxyacetic acid as a linker, to which the first protected amino acid will be introduced, is coupled to the aminoethyl group. Thus, a microtiter plate having wells with a derivatized inner wall is prepared. All these techniques have been widely used in the field of peptide synthesis.

### Example 2: Preparation of a microtiter plate having a derivatized membrane filter placed in its wells

A polypropylene filter and membrane filters of cotton cloth and cellulose paper, each having a thickness of 50 to 60 µm, are respectively derivatized according to the method described elsewhere [J. Eichler et al., Peptide Research, vol. 4, no. 5, 296-307 (1991)]. Specifically, functional groups are introduced to each membrane, and hydroxyphenoxyacetic acid as a linker, to which the first protected amino acid will be introduced, is coupled to these functional groups such as a hydroxy group when the membrane filter is made of cellulose or cotton and an aminoethyl group when the membrane filter is made of a polymer. Thus, a derivatized membrane is prepared. Each of the derivatized membrane is cut to give pieces which fits for the wells in terms of size and number, and each of the pieces is placed in each well of a microtiter plate.

### Example 3: Peptide synthesis

Using the needle for injection/aspiration of reagent, the first protected amino acid solution is delivered from a reagent bottle to a well 2 of a microtiter plate 1 having a derivatized membrane filter as obtained in Example 2 in its wells. The first protected amino acid is added in excess for the size of the membrane filter. Each required amount (e.g., 10-fold excess) of reagents such as PyBOP®(benzotriazol-1-yl-oxy-tris(pyrrolidino)phosphonium hexafluorophosphate), HBTU(2-(1H-benzotriazol-1-yl)-1,1,3,3-tetramethyluronium hexafluorophosphate), HOBt(N-hydroxybenzotriazole) and NMM(N-methylmorpholine) and other reagents used for ordinary peptide synthesis is delivered to the well 2 via a needle 4 connected to the corresponding line. While supplying nitrogen gas from a needle 4 connected to a gas supply line for bubbling the solution in the well, the coupling reaction is carried out. The microtiter plate is vibrated for stirring the solution in the well by vortex-like shaker or inert gas (Argon or N₂) bubbling for a given period of time (about 15 to 30 minutes) to give mixing. After the completion of introduction of the first protected amino acid, the well 2 is thoroughly washed with DMF(dimethylformamide), and in order to remove a protecting group a 2% DBU (1.8-diazabicyclo [5,4,0] undec-7-ene) in DMF, 2% DBU in DMF with 2% piperidine in DMF or 20 - 30% piperidine in DMF is delivered to the well 2 via a needle 4 connected to the corresponding reagent supply line, followed by 10-minute mixing. Washing is conducted in the same manner as above, and an activated and protected amino acid is added for the introduction of the second amino acid, and the same procedure as above is repeated to elongate the peptide chain. The above process is conducted in accordance with the solid-phase peptide synthesis method based on ordinary Fmoc chemistry.

After completion of the sequential introduction of the desired amino acids, Nα-protecting group is removed in the same manner as above, and the well 2 is thoroughly washed with DMF and then with methanol, followed by drying the well and the filter membrane by a blow of an inert gas such as a nitrogen gas and an argon gas via the needle 4 connected to the gas supply line. Then, a cleavage cocktail such as a mixture of TFA, thioanisole and ethanediol, etc. is added to obtain a solution of the desired peptide in the well 2. The solution in the well 2 is transferred to another micro test tube. The solution placed in the test tube is dried by a blow of an inert gas such as a nitrogen gas and an argon gas to reduce its volume, after which dry ether is added and then centrifuged to yield the desired peptide as a residue, or diluted with water/acetonitrile (95:5) and purified by HPLC.

When peptide synthesis is conducted using the peptide synthesizer of the present invention, it is possible to synthesize different peptides in different wells. For example, when a 96-well microtiter plate is used, 96 different peptides can be synthesized concurrently. The peptide synthesizer of the present invention is therefore capable of providing rapid, simultaneous synthesis of a plurality of peptides on a small scale at high efficiency and at low cost.

Also, with respect to the needle 4 for injection/aspiration of reagent, a single needle may be shared by all the wells by moving it over the wells, and it is also possible to equip the peptide synthesizer with the same number of needles as the number of the wells of the microtiter plate used in the present invention.

The present invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

## Claims

1. In an apparatus for peptide synthesis, having a means of repeating a series of treatments consisting of washing, coupling, washing and deprotecting in a stream of an inert gas and being equipped with a needle for injection /aspiration of reagents, the apparatus including a microtiter plate with wells of which inner wall has a linker.

2. The apparatus according to claim 1, wherein the linker is one member selected from the group consisting of hydroxyphenoxyacetic acid, 4-hydroxymethylbenzoic acid, 4-(4-hydroxymethyl-3-methoxyphenoxy)-butyric acid, 4-hydroxymethylphenoxyacetic acid, 3-(4-hydroxymethylphenoxy) propionic acid, 2,4-dimethoxy-4'-hydroxy-benzophenone and p-[(R,S)-α-[1-(9H-fluoren-9-yl)-methoxyformamido]-2,4-dimethoxybenzyl]-phenoxyacetic acid.

3. The apparatus according to claim 1, wherein the linker is phenylacetamidomethyl-derivatized polymer.

4. In an apparatus for peptide synthesis, having a means of repeating a series of treatments consisting of washing, coupling, washing and deprotecting in a stream of an inert gas and being equipped with a needle for injection /aspiration of reagents, the apparatus including a microtiter plate having a membrane filter, which has a linker, placed in the wells.

5. The apparatus according to claim 4, wherein the linker is one member selected from the group consisting of hydroxyphenoxyacetic acid, 4-hydroxymethylbenzoic acid, 4-(4-hydroxymethyl-3-methoxyphenoxy)-butyric acid, 4-hydroxymethylphenoxyacetic acid, 3-(4-hydroxymethylphenoxy) propionic acid, 2,4-dimethoxy-4'-hydroxy-benzophenone and p-[(R,S)-α-[1-(9H-fluoren-9-yl)-methoxyformamido]-2,4-dimethoxybenzyl]-phenoxyacetic acid.

6. The apparatus according to claim 4, wherein the linker is phenylacetamidomethyl-derivatized polymer.

7. A microtiter plate for use in a reaction based on molecular recognition, wherein a peptide synthesized using the apparatus of claim 3 or 6 is immobilized thereon.
